# EUROPEAN PATENT APPLICATION

(11) **EP 4 083 052 A1**
(43) Date of publication of application: **02.11.2022**
(21) Application number: 21171206.2
(22) Date of filing: 29.04.2021
(51) Int. Cl.: C07K 5/06, A61P 35/00

(54) **PEPTIDE CONJUGATES OF MELPHALAN FOR THE TREATMENT OF CANCER**

(71) Applicant: BKmdcl AB, 129 38 Hägersten (SE)
(72) Inventor: KLASSON, Björn-Olof, 129 38 HÄGERSTEN (SE)
(74) Representative: Brann AB

(57) **Abstract**

The present invention relates to a compound represented by formula (I): 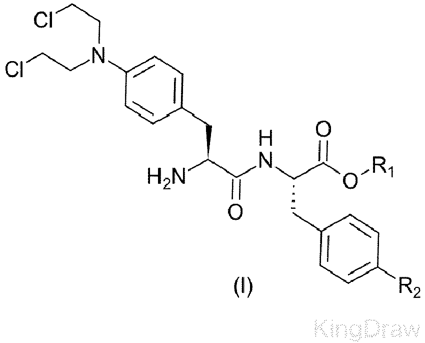 wherein:
R₁ is H, C₁-C₁₀ alkyl, C₂-C₁₀ alkenyl, C₃-C₇cycloalkyl, C₃-C₇cycloalkyl C₁-C₃ alkyl, benzyl, or phenyl, any of which is optionally substituted with 1, 2 or 3 groups, each independently comprising halogen;
R₂ is Br, CI, I, CN, methyl, ethyl, n-propyl, propan-2-yl, cyclopropyl, 1-methyl-1-cyclopropyl, cyclobutyl, 1-methyl-1-cyclobutyl, cyclopentyl, 1-methyl-1-cyclopentyl, Si(CH₃)₃, C(CH₃)₃ or CXF₂, CX₂F or CX₃ where X is Cl or Br as well as pharmaceutically acceptable salts and/or solvates thereof.

The compound may be used as a medicament; or in the treatment of cancer.

Further, the invention relates to a method of treating a patient suffering from cancer by administering a compound according to the invention.

## Description

### TECHNICAL FIELD

The present invention relates to peptide conjugates of melphalan and derivatives thereof which are useful in the treatment of cancers in humans and other warm blooded animals, in particular hematological malignancies such as Multiple Myeloma and lymphomas, but also useful for treating solid tumors. The invention further relates to compositions and combinations comprising these compounds, and methods for their use in the treatment of cancers.

### BACKGROUND OF THE INVENTION

Globally, multiple myeloma affected 488,000 people and resulted in 101,100 deaths in 2015. In the United States, multiple myeloma developed in 6.5 per 100,000 people per year. Without treatment, survival time is, typically, seven months. With current treatments, survival time is usually 4-5 years. The five-year survival rate with current treatment is about 54%. Melphalan has been used for the treatment of malignancies such as multiple myeloma and lymphomas for more than fifty years^{1,2}. Melphalan has also been used to treat cancers such as breast cancer, ovarian cancer and neuroblastoma. The usefulness of melphalan, when given alone or in combination with other cytotoxic drugs, is limited because of off-target toxicities of melphalan, low concentration of compound reaching and entering malignant cells and the development of drug resistance. These restrictions are reported to have been partly overcome by the introduction a prodrug component to melphalan where melphalan has been conjugated, via an amide bond, to *para*-fluoro-L-phenylalanine ethyl ester resulting in L-melphalanyl-p-L-fluorophenylalanine ethyl ester which has advanced into clinical development³. Aminopeptidases, such as aminopeptidase N are often over expressed in tumor cells and are able to cleave the melphalan conjugate (L-melphalanyl-p-L-fluorophenylalanine ethyl ester) and thereby releasing melphalan in tumor cells (Scheme 1). The conjugate L-melphalanyl-p-L-fluorophenylalanine ethyl ester is relatively more lipophilic than melphalan and passes more readily cell membranes than does melphalan. Melphalan, when being released in tumor cells and as being less lipophilic, is thereby trapped in the tumor cells resulting in accumulation of melphalan in tumor cells over-expressing aminopeptidase.

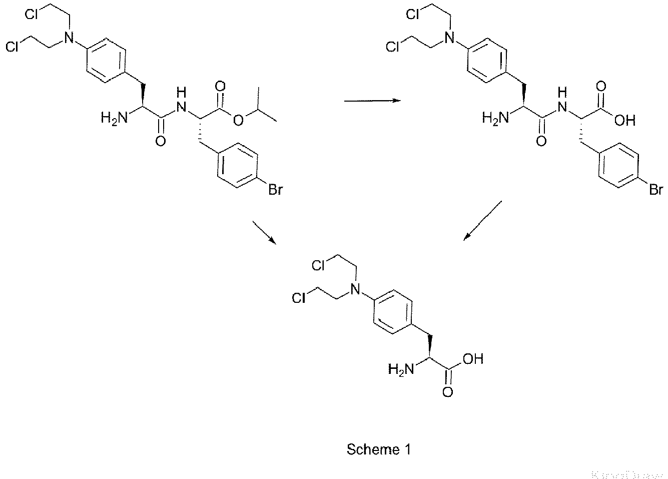

Melphalan is a "mustard-type" (bis(2-chloroethyl)amine) alkylating agent, and is thereby inherently toxic not only to the malignant cells.

By introducing
g other peptide conjugates, than what has been described above, to the melphalan, better and therapeutically relevant concentrations of melphalan can be achieved in tumor cells and reducing concentrations in non-cancer cells. The intracellular and intercellular hydrolysis of the melphalan conjugate to melphalan is depicted in scheme 1 below.

Prodrug conjugates of melphalan have been described in WO2001096367.

### SUMMARY OF THE INVENTION

The present invention relates to a compound represented by formula (I): 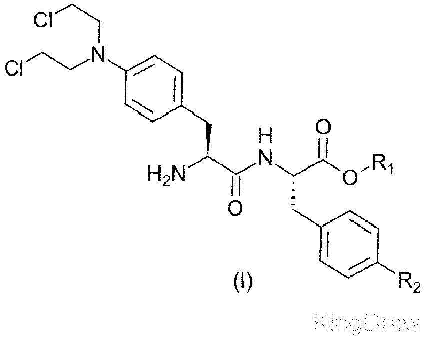 wherein:
R₁ is H, C₁-C₁₀ alkyl, C₂-C₁₀ alkenyl, C₃-C₇CyCloalkyl, C₃-C₇cycloalkyl C₁-C₃ alkyl, benzyl, or phenyl, any of which is optionally substituted with 1, 2 or 3 groups, each independently comprising halogen;
R₂ is Br, Cl, I, CN, methyl, ethyl, n-propyl, propan-2-yl, cyclopropyl, 1-methyl-1-cyclopropyl, cyclobutyl, 1-methyl-1-cyclobutyl, cyclopentyl, 1-methyl-1-cyclopentyl, Si(CH₃)₃, C(CH₃)₃ or CXF₂, CX₂F or CX₃ where X is Cl or Br as well as pharmaceutically acceptable salts and/or solvates.

Further, the invention relates to a compound as defined above for use as a medicament, such as in the treatment of cancer.

Thus, the invention also relates to a method of treating a patient suffering from cancer, such as a cancer selected from the group consisting of lung cancer, breast cancer, ovarian cancer, testicular cancer, multiple myeloma and malignant melanoma, which method comprises the step of administering a pharmaceutically efficient dose of a compound as defined above to said patient.

Finally, the invention relates to a pharmaceutical preparation, which comprises a compound as defined above in combination with a pharmaceutically acceptable carrier.

### DESCRIPTION OF THE INVENTION

The present invention relates to novel peptide conjugates of phenylalanine mustard comprising (2S)-2-amino-3-{4-[bis(2-chloroethyl)amino]phenyl}propionic acid (melphalan) covalently linked, via an amide bond, to an amino acid derivative.

In one embodiment, the present invention provides compounds represented by formula (I): 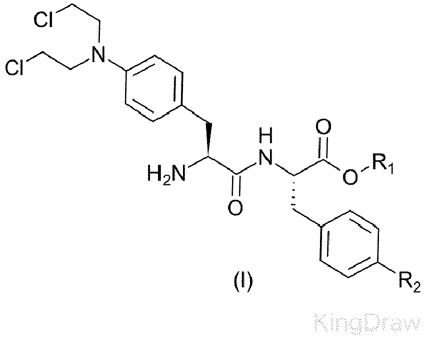 wherein:
R₁ is H, C₁-C₁₀ alkyl, C₂-C₁₀ alkenyl, C₃-C₇CyCloalkyl, C₃-C₇cycloalkyl C₁-C₃ alkyl, benzyl, or phenyl, any of which is optionally substituted with 1, 2 or 3 groups, each independently comprising halogen;
R₂ is Br, Cl, I, CN, methyl, ethyl, n-propyl, propan-2-yl, cyclopropyl, 1-methyl-1-cyclopropyl, cyclobutyl, 1-methyl-1-cyclobutyl, cyclopentyl, 1-methyl-1-cyclopentyl, Si(CH₃)₃, C(CH₃)₃ or CXF₂, CX₂F or CX₃ where X is Cl or Br as well as pharmaceutically acceptable salts and/or solvates thereof.

In another aspect, the invention provides compounds represented by formula (I) wherein:
R₁ is methyl, ethyl, proanpyl, propan-2-yl, pentan-3-yl, cyclopropylmethyl, 2-methylpropan-1-yl, 2-ethylbutan-1-yl, 2-propylpentan-1-yl, (2S)-butan-2-yl, (2R)-butan-2-yl, (2S)-pentan-2-yl, (2R)-pentan-2-yl, (2R)-hexan-2-yl, (2S)-hexan-2-yl, cyclopropyl, cyclobutyl, cyclopentyl or cyclohexyl;
R₂ is Br, Cl, I, CN, methyl, ethyl, n-propyl, propan-2-yl, cyclopropyl, 1-methyl-1-cyclopropyl, cyclobutyl, 1-methyl-1-cyclobutyl, cyclopentyl, 1-methyl-1-cyclopentyl, Si(CH₃)₃, C(CH₃)₃ or CXF₂, CX₂F or CX₃ where X is Cl or Br as well as pharmaceutically acceptable salts and/or solvates thereof.

In further aspect, the invention provides compounds represented by formula (I) wherein:
R₁ is propan-2-yl, pentan-3-yl, 2-methylpropan-1-yl, 2-ethylbutan-1-yl, 2-propylpentan-1-yl, (2S)-butan-2-yl, (2R)-butan-2-yl, (2S)-pentan-2-yl, (2R)-pentan-2-yl, cyclopropyl or cyclopentyl;
R₂ is Br, Cl, I, CN, methyl, ethyl, n-propyl, propan-2-yl, cyclopropyl, 1-methyl-1-cyclopropyl, cyclobutyl, 1-methyl-1-cyclobutyl, cyclopentyl, 1-methyl-1-cyclopentyl, Si(CH₃)₃, C(CH₃)₃ or CXF₂, CX₂F or CX₃ where X is Cl or Br as well as pharmaceutically acceptable salts and/or solvates thereof.

In further aspect, the invention provides compounds represented by formula (I) wherein:
R₁ is propan-2-yl, pentan-3-yl, (2S)-pentan-2-yl or (2R)-pentan-2-yl;
R₂ is Br, Cl, CN, 1-methyl-1-cyclopropyl, cyclopropyl, Si(CH₃)₃, or C(CH₃)₃.

In further aspect, the invention provides compounds represented by formula (I) wherein:
R₁ is pentan-3-yl or propan-2-yl;
R₂ is Br, Si(CH₃)₃ or C(CH₃)₃.

The compounds of formula (I) may optionally be provided in the form of a pharmaceutically acceptable salt and/or solvate. In one embodiment the compound of the invention is provided in the form of a pharmaceutically acceptable salt. In a second embodiment the compound of the invention is provided in the form of a pharmaceutically acceptable solvate. In a third embodiment the compound of the invention is provided in its free form.

The compounds of the invention may be used as first line or second line or third line treatment, either alone or in combination with other drugs or concomitant or sequenced with radiotherapy, as a method of treating a patient suffering from cancer. Such cancer may be selected from the group consisting of lung cancer, breast cancer, ovarian cancer, testicular cancer, multiple myeloma and malignant melanoma, and the method may comprise the step of administering a pharmaceutically efficient dose of a compound of the invention.

As compared to melphalan, the compounds of the invention can be shown to be more potent, with increased therapeutic window.

The derivatives of the invention can be synthesized from N-tert-butoxycarbonyl (N-Boc) protected melphalan. Coupling of N-Boc protected melphalan to the amino acid ester derivatives can be performed in DMF as solvent and using reagents such as 1-Hydroxybenzotriazole, HOBt; N-methylmorpholine, NMM; and/or 1-[3-(dimethylamino)propyl]-3-ethylcarbodiimide hydrochloride, EDC; or (benzotriazol-1-yloxy) -tripyrrolidino-phosphoniumhexafluorophosphate, PyBOP; and triethyl amine. After coupling, hydrolysis of the N-Boc group can be performed in HCI and diethyl ether or in TFA/TIS/H2O.

### GENERAL SYNTHESES METHODS

Compounds of the invention may be prepared by a variety of methods e.g. as depicted in the illustrative synthetic scheme 2 and described below. The starting materials and reagents used are available from commercial suppliers or can be prepared according to literature procedures using methods well known to those skilled in the art. Scheme 2 illustrates a general route to compounds in formula (I).

### SYNTHESIS DESCRIPTION (Scheme 2)

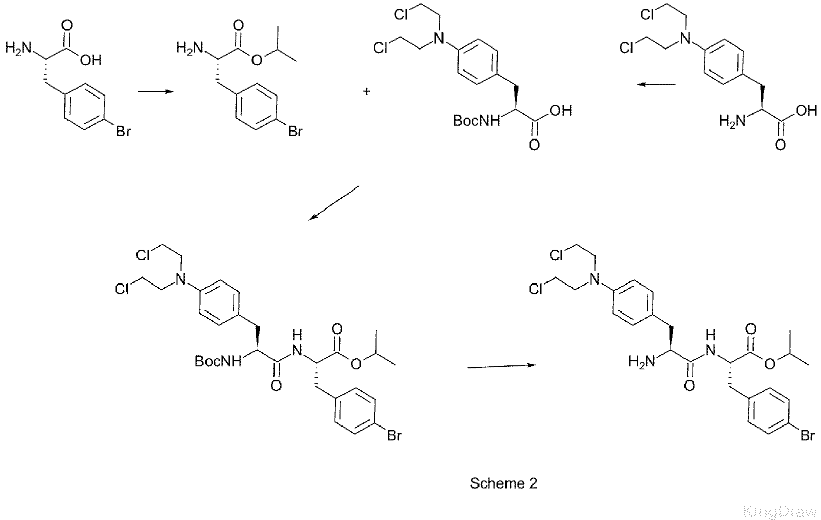

### Para substituted amino acid 2-propanol ester:

Para bromo phenylalanine, (S)-2-Amino-3-(4-(bromo)phenyl)propanoic acid, can be heated in an HCl/2-propanol solution and then concentrated to dryness. (As alternative the N-Boc protected amino acid can be coupled to the alcohol using dimethylaminopyridine/dicyclohexylcarbodiimide as coupling reagent, followd by N-Boc deprotection with HCI). By substituting 2-propanol for other primary or secondary alcohols, the corresponding esters can be obtained.

### N-Boc melphalan:

The formation of N-Boc melphalan is conducted under either aqueous or anhydrous conditions, by reaction with a base and di-tert-butyl dicarbonate (Boc anhydride). Adding di-tert-butyl dicarbonate (2 equiv.) to a mixture of melphalan (1 equiv.) and sodium bicarbonate (4 equiv.) in water/THF and stirring at room temperature over night and subsequently extracting the mixture with dichloromethane followed by concentration to dryness will render the N-Boc melphalan.

Peptide coupling of the amino acid ester to N-Boc melphalan and removing the N-Boc group:
Diisopropylethylamine (5 equiv.) is added to a solution of HATU (1-[Bis(dimethylamino)methylene]-1H-1,2,3-triazolo[4,5-b]pyridinium 3-oxide hexafluorophosphate) (3.3 equiv.) and N-Boc melphalan (1 equiv.) in dimethylformamide (DMF). After 5 min the para substituted amino acid esters (1 equiv.) in DMF is added. After 1 day the mixture is partitioned between water and dichloromethane. The organic layer is separated and dried with magnesium sulfate, concentrated and purified by chromatography. The solid compound is dissolved in a cleavage cocktail TFA/TIS/H₂O (95/2.5/2.5, v/v) at 0°C. After 3 h the mixture is concentrated and the residue purified by chromatography.

### CELL CYTOTOXICITY ASSAY

Cells chosen from: MM.1S, lymphoma U-937 GBT, myeloma RPMI 8226, U266, NCI-H929, or any other lymphoma cell line, small cell lung cancer NCI-H69, leukemia CCRF-CEM, (10000 cells/well in a 96-well format) are seeded 24 hours prior to the addition of a compound of the invention (serially diluted from 100 µM), and allowed to incubate for 3 days at 37° C. A medium only control is used to determine the minimum absorbance value and an untreated cell value. At the end of the growth period, CellTitre-Glo reagent, XTT or MTT dye is added to each well. After 30 min the luminiscence signal, or the absorbance at 450 nm with a reference wavelength of 600 nM, is read using the medium only control wells as blanks. The 50% inhibition value (CC₅₀) is determined by comparing the degree of inhibition (compared to cell control) and plotted against compound concentration. Results from the dilution series is fitted to a sigmoidal dose-response curve.

### References:

1. J Clin Oncol. 1995 Jul;13(7):1786-99.
2. J. Clin. Oncol., 2: 1289-1299, 1984.
3. J. Clin. Med. 2020, 9, 3120; doi:10.3390/jcm9103120.

## Claims

1. A compound represented by formula (I): 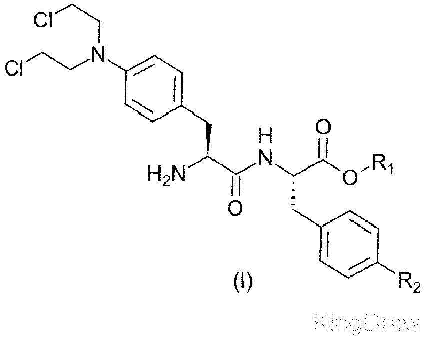 wherein:
R₁ is H, C₁-C₁₀ alkyl, C₂-C₁₀ alkenyl, C₃-C₇CyCloalkyl, C₃-C₇cycloalkyl C₁-C₃ alkyl, benzyl, or phenyl, any of which is optionally substituted with 1, 2 or 3 groups, each independently comprising halogen;
R₂ is Br, Cl, I, CN, methyl, ethyl, n-propyl, propan-2-yl, cyclopropyl, 1-methyl-1-cyclopropyl, cyclobutyl, 1-methyl-1-cyclobutyl, cyclopentyl, 1-methyl-1-cyclopentyl, Si(CH₃)₃, C(CH₃)₃ or CXF₂, CX₂F or CX₃ where X is Cl or Br as well as pharmaceutically acceptable salts and/or solvates thereof.

2. A compound according to claim 1 for use as a medicament.

3. A compound according to claim 1 for use in treating cancer.

4. A compound for use according to claim 3, which includes treatment of tumor diceases selected from the group consisting of lung cancer, breast cancer, ovarian cancer, testicular cancer, multiple myeloma and malignant melanoma.

5. A compound according to claim 1, or a compound for use according to any one of claims 2-4, wherein:
R₁ is methyl, ethyl, proanpyl, propan-2-yl, pentan-3-yl, cyclopropylmethyl, 2-methylpropan-1-yl, 2-ethylbutan-1-yl, 2-propylpentan-1-yl, (2S)-butan-2-yl, (2R)-butan-2-yl, (2S)-pentan-2-yl, (2R)-pentan-2-yl, (2R)-hexan-2-yl, (2S)-hexan-2-yl, cyclopropyl, cyclobutyl, cyclopentyl or cyclohexyl; and
R₂ is Br, Cl, I, CN, methyl, ethyl, n-propyl, propan-2-yl, cyclopropyl, 1-methyl-1-cyclopropyl, cyclobutyl, 1-methyl-1-cyclobutyl, cyclopentyl, 1-methyl-1-cyclopentyl, Si(CH₃)₃, C(CH₃)₃ or CXF₂, CX₂F or CX₃ where X is Cl or Br.

6. A compound or a compound for use according claim 5, wherein:
R₁ is propan-2-yl, pentan-3-yl, 2-methylpropan-1-yl, 2-ethylbutan-1-yl, 2-propylpentan-1-yl, (2S)-butan-2-yl, (2R)-butan-2-yl, (2S)-pentan-2-yl, (2R)-pentan-2-yl, cyclopropyl or cyclopentyl; and
R₂ is Br, Cl, I, CN, methyl, ethyl, n-propyl, propan-2-yl, cyclopropyl, 1-methyl-1-cyclopropyl, cyclobutyl, 1-methyl-1-cyclobutyl, cyclopentyl, 1-methyl-1-cyclopentyl, Si(CH₃)₃, C(CH₃)₃ or CXF₂, CX₂F or CX₃ where X is Cl or Br.

7. A compound or a compound for use according to claim 6, wherein:
R₁ is propan-2-yl, pentan-3-yl, (2S)-pentan-2-yl or (2R)-pentan-2-yl; and
R₂ is Br, Cl, CN, 1-methyl-1-cyclopropyl, cyclopropyl, Si(CH₃)₃, or C(CH₃)₃.

8. A compound or a compound for use according to claim 7, wherein:
R₁ is pentan-3-yl or propan-2-yl; and
R₂ is Br, Si(CH₃)₃ or C(CH₃)₃.

9. A method of treating a patient suffering from cancer, such as a cancer selected from the group consisting of lung cancer, breast cancer, ovarian cancer, testicular cancer, multiple myeloma and malignant melanoma, which method comprises the step of administering a pharmaceutically efficient dose of a compound as defined in any one of claims 1 or 5-8 to said patient.

10. A method according to claim 9, wherein said treatment is administered by central infusion, intravenous infusion or per-oral.

11. A pharmaceutical preparation, which comprises a compound as defined in any one of claims 1 or 5-8 in combination with a pharmaceutically acceptable carrier.
